# EUROPEAN PATENT APPLICATION

(11) **EP 4 599 871 A1**
(43) Date of publication of application: **13.08.2025**
(21) Application number: 23876592.9
(22) Date of filing: 07.10.2023
(51) Int. Cl.: A61M 11/00, A61M 16/00, A24F 40/50

(54) **CONNECTOR, CONTROLLER, ATOMIZER, AND ELECTRONIC ATOMIZATION SYSTEM**

(30) Priority: 09.10.2022 CN 202211229585
(71) Applicant: Shenzhen Moore Vaporization Health & Medical Technology Co., Ltd., Shenzhen, Guangdong 518102 (CN)
(72) Inventor: LYU, Yong, Shenzhen, Guangdong 518102 (CN); LI, Jun, Shenzhen, Guangdong 518102 (CN); CHENG, Shiyi, Shenzhen, Guangdong 518102 (CN); XIE, Xitian, Shenzhen, Guangdong 518102 (CN)
(74) Representative: Winter, Brandl - Partnerschaft mbB
(86) International application number: PCT/CN2023/123250
(87) International publication number: WO 2024/078390

(57) **Abstract**

This application provides a connector, a controller, an atomizer and an electronic atomization system. The connector includes a first body portion and a plurality of first electrical connection terminals arranged in the first body portion. The plurality of first electrical connection terminals are configured to be electrically connected to the atomizer and a control component in the controller, so as to enable the control component to control the atomizer to work through the plurality of first electrical connection terminals, and detect the temperature of the atomizer, thereby diversifying functions of an electrical connection interface between the controller and the atomizer, and implementing the high system integration degree.

## Description

### CROSS-REFERENCE TO RELATED APPLICATIONS

This application claims priority to Chinese Patent Application No. 202211229585.5, filed on October 9, 2022, which is incorporated by reference in its entirety.

### TECHNICAL FIELD

This application relates to the technical field of atomization, and in particular to a connector, a controller, an atomizer and an electronic atomization system.

### BACKGROUND

An existing electronic atomization system for medical use generally includes an atomizer and a controller. The controller is configured to control the atomizer to work. An atomization component in the atomizer is configured to atomize a liquid medium, such as a medical solution or a nutrient solution, introduced onto the atomization component, so as to generate an aerosol for inhalation treatment of a patient. Because an atomization process is a physical process, the property of the liquid is not changed. Thus, the existing electronic atomization system is widely applied to the field of medical instruments.

However, an electrical connection interface between the controller and the atomizer in the existing electronic atomization system can only meet a control function, and thus has a relatively single function and is difficult to adapt to the development trend of electronic atomization systems.

### SUMMARY

This application provides a connector, a controller, an atomizer and an electronic atomization system, which can solve the problem that an electrical connection interface between the controller and the atomizer has a relatively single function.

To solve the above problem, this application provides a technical solution as follows: Provided is a connector, applied to an electronic atomization system, including: a first body portion and a plurality of first electrical connection terminals arranged in the first body portion. The plurality of first electrical connection terminals are configured to be electrically connected to an atomizer and a control component in a controller, so as to enable the control component to control the atomizer to work through the plurality of first electrical connection terminals, and detect the temperature of the atomizer.

**In** an embodiment, the plurality of first electrical connection terminals include a first control terminal set and a first detection terminal set; the first control terminal set includes a positive control terminal and a negative control terminal; the first detection terminal set includes a positive detection terminal and a negative detection terminal; the shape of the radial cross section of the first body portion is an axisymmetric figure, and the positive control terminal and the negative control terminal are symmetrically arranged along the symmetric axis of the axisymmetric figure; and the positive detection terminal and the negative detection terminal are symmetrically arranged along the symmetric axis of the axisymmetric figure.

In an embodiment, the plurality of first electrical connection terminals further include a first charge terminal set, and the first charge terminal set includes a positive charge terminal and a negative charge terminal.

In an embodiment, the positive charge terminal and the negative charge terminal are separately arranged connection terminals; or the positive charge terminal is a separately arranged connection terminal, and the negative control terminal or the negative detection terminal is used as the negative charge terminal.

In an embodiment, the shape of the radial cross section of the first body portion is a non-centrosymmetric figure.

In an embodiment, the first body portion has a plurality of mounting holes, and the mounting holes are through holes arranged along the axial direction of the first body portion; the plurality of first electrical connection terminals are arranged in the plurality of mounting holes in a one-to-one correspondence manner; and one end of the plurality of first body portions configured to be connected to the atomizer is a first end, and the distance between each of the plurality of first electrical connection terminal and the end surface of the first end of the first body portion is 2 mm to 5 mm.

In an embodiment, the first body portion is a groove body, the plurality of first electrical connection terminals are arranged spaced away in the groove body and are fixed to the bottom wall of the groove body, and the distance between the plurality of first electrical connection terminals and the plane at which the groove opening of the groove body is located is 2 mm to 5 mm.

To solve the above problem, this application provides another technical solution as follows: Provided is a controller, including: a housing having a first accommodating cavity; a control component, accommodated in the first accommodating cavity; a power supply component, electrically connected to the control component; and a connector, arranged on the housing and electrically connected to the control component, the connector being further configured to be electrically connected to the atomizer, where the connector is the connector of any one of the above items.

In an embodiment, the housing includes the annular side wall, the top wall and the bottom wall, and the annular side wall, the top wall and the bottom wall cooperate to define the first accommodating cavity; and a first body portion is arranged on the outer side of the top wall and is integrally formed and designed with the top wall, or the first body portion is arranged on the outer side of the bottom wall and is integrally formed and designed with the bottom wall.

In an embodiment, a buckle structure is arranged on a first body portion or the housing, and the buckle structure is configured to fix the atomizer when the connector is electrically connected to the atomizer.

To solve the above problem, this application provides still another technical solution as follows: Provided is an atomizer, including: a shell having a second accommodating cavity; an atomization component, accommodated in the second accommodating cavity; and a second connection portion arranged on the shell and configured to be plugged with a connector, and the second connection portion including a second body portion and a plurality of second electrical connection terminals arranged on the second body portion and electrically connected to the atomization component, and the plurality of second electrical connection terminals being configured to be electrically connected to a plurality of first electrical connection terminals on the connector.

To solve the above problem, this application provides yet another technical solution as follows: Provided is an electronic atomization system, including an atomizer, the atomizer being the atomizer of any one of the above items; and a controller, the controller being the controller of any one of the above items.

Different from the prior art, according to the connector, the controller, the atomizer and the electronic atomization system provided in this application, the connector includes a first body portion and a plurality of first electrical connection terminals arranged in the first body portion. The plurality of first electrical connection terminals are configured to be electrically connected to the atomizer and the control component in the controller, so as to enable the control component to control the atomizer to work through the plurality of first electrical connection terminals, and detect the temperature of the atomizer, thereby diversifying functions of an electrical connection interface between the controller and the atomizer, and implementing the high integration degree.

### BRIEF DESCRIPTION OF THE DRAWINGS

To describe the technical solutions in the embodiments of this application more clearly, the following briefly introduces the accompanying drawings required for describing the embodiments. Apparently, the accompanying drawings in the following description show only some embodiments of this application. Those of ordinary skill in the art may still derive other accompanying drawings from these accompanying drawings without creative efforts.
FIG. 1 is a schematic structural diagram of an embodiment of an electronic atomization system provided in this application;
FIG. 2 is a schematic structural diagram of a controller shown in FIG. 1;
FIG. 3 is a schematic structural diagram of the controller shown in FIG. 2 after a part of a housing is removed;
FIG. 4 is a cross-sectional view of the controller shown in FIG. 2 along a line A-A;
FIG. 5 is a structural enlarged view of a region A in FIG. 4;
FIG. 6 is a structural enlarged view of a region A in another embodiment of a controller provided in this application;
FIG. 7 is a schematic structural diagram of a connector from one perspective in another embodiment of a controller provided in this application;
FIG. 8 is a schematic structural diagram of a connector from one perspective in the controller shown in FIG. 2;
FIG. 9 is a schematic structural diagram of an atomizer shown in FIG. 1;
FIG. 10 is a schematic structural diagram of an atomizer shown in FIG. 8 from another perspective; and
FIG. 11 is a structural exploded view of an embodiment of an atomization component provided in this application.

### DETAILED DESCRIPTION

The following clearly and completely describes technical solutions in embodiments of this application with reference to the accompanying drawings in the embodiments of this application. It may be understood that specific embodiments described herein are only used to explain this application, but not to limit this application. In addition, it should also be noted that for ease of description, the accompanying drawings only show parts relevant to this application rather than the entire structure. All other embodiments obtained by those of ordinary skill in the art based on the embodiments of this application without creative efforts shall fall within the protection scope of this application.

In the above description of this specification, unless explicitly specified and limited otherwise, terms such as "fixing", "mounting", "connected", or "connection" should be understood in a broad sense. For example, the term "connection" may be fixed connection, detachable connection or integrated connection; may be mechanical connection or electrical connection; or may be direct connection, indirect connection through an intermediate medium, or internal communication between two elements or interaction relationship between two elements. Therefore, unless otherwise limited in this specification, those skilled in the art can understand the specific meanings of the above terms in this application according to specific situations.

The terms "first" and "second" in this application are merely intended for the purpose of description, and shall not be understood as indicating or implying relative significance or implicitly indicating the number of indicated technical features. Therefore, the feature restricted by "first" or "second" may explicitly indicate or implicitly include at least one of such features. In addition, the terms "include", "have" and any variants thereof are intended to cover non-exclusive inclusions. For example, processes, methods, systems, products, or devices that include a series of steps or units are not limited to the listed steps or units, and instead, further optionally include steps or units that are not listed, or further optionally include other steps or units that are intrinsic to these processes, methods, products, or devices.

The "embodiments" mentioned in this specification mean that specific features, structures or characteristics described with reference to the embodiments may be included in at least one embodiment of this application. The appearances of the phrase in various positions of the specification do not necessarily refer to the same embodiment, nor a separate or an alternative embodiment that is mutually exclusive with other embodiments. Those skilled in the art explicitly or implicitly understand that the embodiments described in this specification may be combined with other embodiments.

The following describes this application in detail with reference to the accompanying drawings and embodiments.

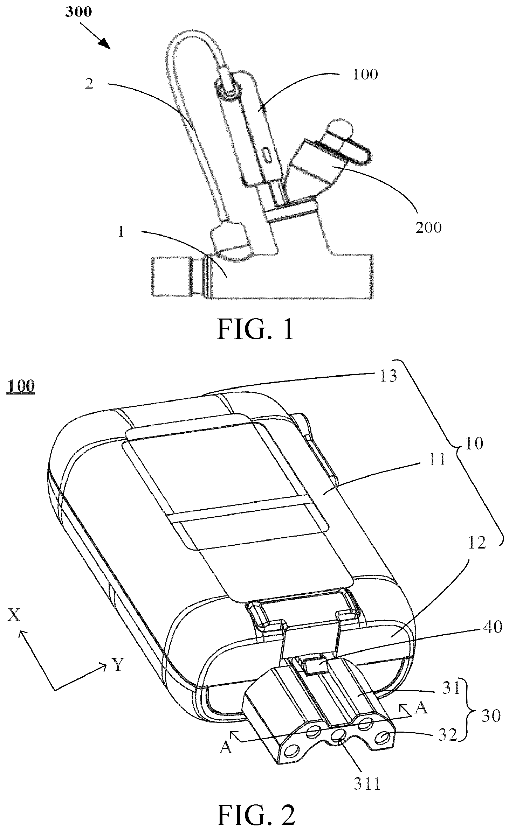

Referring to FIG. 1, FIG. 1 is a schematic structural diagram of an embodiment of an electronic atomization system provided in this application. Specifically, this application provides an electronic atomization system 300 for medical use, including a controller 100 and an atomizer 200 that are electrically connected to each other. An atomization component is arranged in the atomizer 200. The atomization component may be an ultrasonic atomization component, an electric heating atomization component, an air compression atomization component, or a mechanical vibration atomization component. The atomization component is configured to atomize an aerosol-forming medium such as a medical solution or a nutrient solution under the control of the controller.

In this embodiment, the electronic atomization system 300 further includes a three-way tube 1 that communicates with a vapor outlet 201 of the atomizer 200, and the three-way tube 1 is configured to export the atomization gas generated by the atomizer 200 to a respirator for use by a patient.

Further, an air pressure sensing module is also arranged in the controller 100, and the controller 100 communicates with the three-way tube 1 through an air pressure sensing connection pipeline 2 and detects the air pressure in the three-way tube 1. The controller 100 controls an atomization operation of the atomizer 200 based on an air pressure signal detected by the air pressure sensing module.

Specifically, when the air pressure sensing module detects that the respirator sends air under positive pressure and the patient inhales air, the corresponding air pressure signal is sent to the controller 100, and the controller 100 controls the atomizer 200 to start the atomization operation for administration to the patient; and when the air pressure sensing module detects that the respirator stops sending air and the patient exhales air, the corresponding air pressure signal is sent to the controller 100, and the controller 100 controls the atomizer 200 to stop the atomization operation, thereby preventing ineffective administration and saving medication.

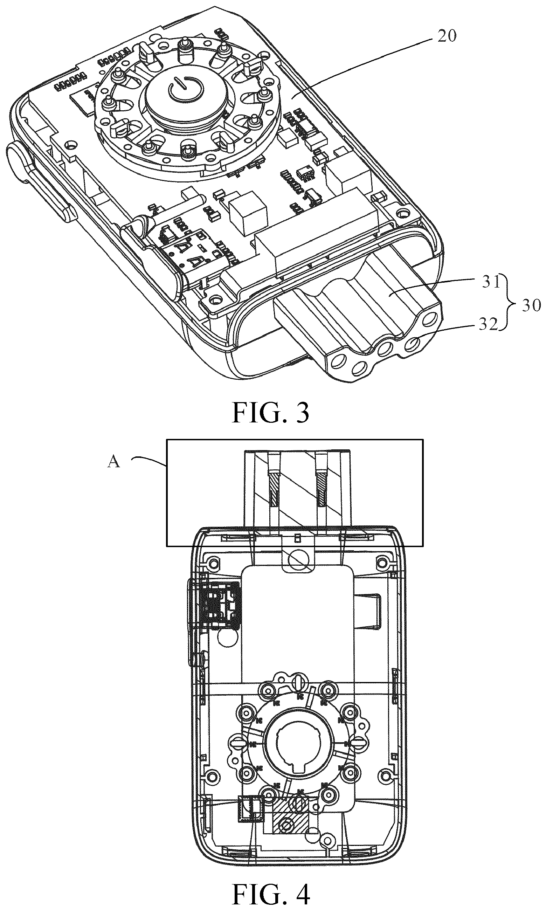

Referring to FIG. 2 and FIG. 3, FIG. 2 is a schematic structural diagram of an embodiment of a controller shown in FIG. 1; and FIG. 3 is a schematic structural diagram of the controller shown in FIG. 2 after a part of a housing is removed.

The controller 100 includes a housing 10, a control component 20, a power supply component (not shown in the figures), and a connector 30. Specifically, the housing 10 has a first accommodating cavity (not shown in the figure); the control component 20 and the power supply component are electrically connected and accommodated in the first accommodating cavity; the connector 30 is arranged on the housing 10; and the connector 30 includes a first body portion 31 and a plurality of first electrical connection terminals 32, where the plurality of first electrical connection terminals 32 are arranged on the first body portion 31 and are electrically connected to the control component 20, and the plurality of first electrical connection terminals 32 are further configured to be electrically connected to the atomizer 200, so as to enable the control component 20 to control the atomizer 200 to work through the plurality of first electrical connection terminals 32, and implement other corresponding functions.

The connector 30 may be detachably connected to the housing 10. For example, the connector 30 is fixedly connected to the housing 10 by way of buckling, bonding, threaded connection, or the like. Alternatively, the connector 30 and the housing 10 are integrally formed and designed. This is not limited herein.

The power supply component may be a mobile power supply component or a wired power supply component. Specifically, when the power supply component is a mobile power supply component, the mobile power supply component is detachably arranged in the first accommodating cavity; and when the power supply component is a wired power supply component, the wired power supply component is fixedly arranged in the first accommodating cavity and may be electrically connected to an external power socket through a connecting wire, so as to charge the wired power supply component.

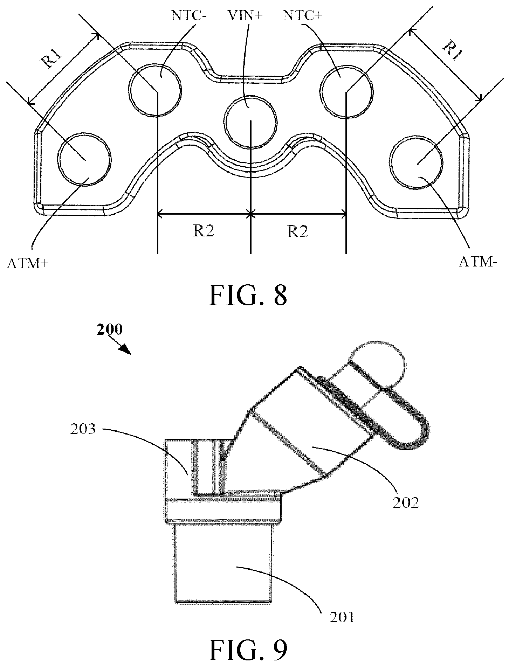

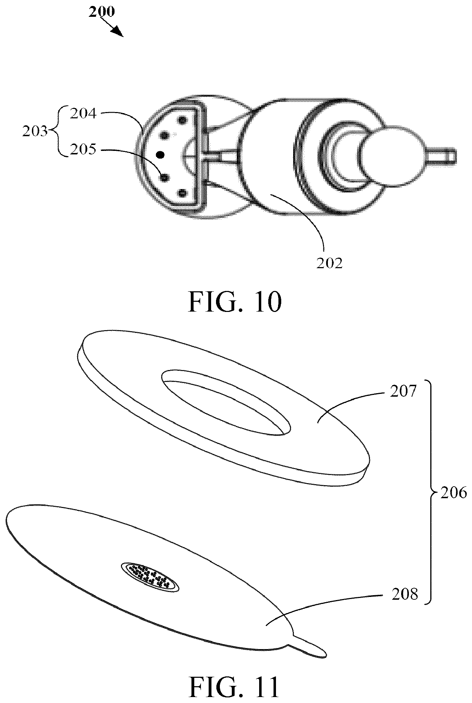

Referring to FIG. 9 and FIG. 10, FIG. 9 is a schematic structural diagram of the atomizer shown in FIG. 1; and FIG. 10 is a schematic structural diagram of the atomizer shown in FIG. 8 from another perspective.

The atomizer 200 includes a shell 202, an atomization component (not shown in the figures), and a second connection portion 203. Specifically, the shell 202 has a second accommodating cavity (not shown in the figures); the atomization component is accommodated in the second accommodating cavity; and the second connection portion 203 is arranged on the shell 202 and is configured to be plugged with the connector 30, so as to implement electrical connection between the controller 100 and the atomizer 200. The second connection portion 203 includes a second body portion 204 and a plurality of second electrical connection terminals 205. The plurality of second electrical connection terminals 205 are arranged on the second body portion 204 and are electrically connected to the atomization component. The plurality of second electrical connection terminals 205 are further configured to be correspondingly electrically connected to the plurality of first electrical connection terminals 32 on the controller 100, so as to enable the control component 20 to control the atomization component to work through the plurality of first electrical connection terminals 32 and the plurality of second electrical connection terminals 205.

In some embodiments, the control component 20 may further detect the temperature, working voltage, working current and/or working power of the atomizer 200 through the plurality of first electrical connection terminals 32 and the plurality of second electrical connection terminals 205; and the atomizer 200 and/or other external charge devices may charge the controller 100 through the plurality of first electrical connection terminals 32.

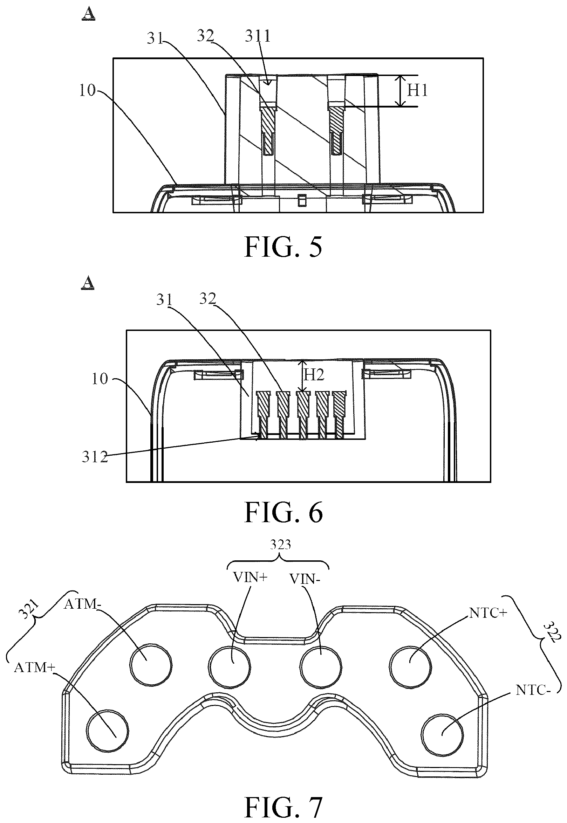

Referring to FIG. 7, FIG. 7 is a schematic structural diagram of a connector from one perspective in another embodiment of a controller provided in this application. In this application, the plurality of first electrical connection terminals 32 at least include a first control terminal set 321 and a first detection terminal set 322. The control component 20 outputs an atomization drive signal through the first control terminal set 321 to drive the atomizer 200 to work, and detects the temperature of the atomizer 200 through the first detection terminal set 322, thereby preventing the atomizer 200 from stopping working or causing potential safety hazards due to an excessively high temperature during working.

In this application, the plurality of second electrical connection terminals 205 at least include a second control terminal set (not shown in the figure) and a second detection terminal set (not shown in the figure). The atomization component receives the atomization drive signal outputted by the controller 100 through the second control terminal set for atomization, and outputs a temperature signal of the atomizer 200 through the second detection terminal set to the controller 100.

Specifically, the first control terminal set 321 includes a positive control terminal ATOM+ and a negative control terminal ATOM-; and the first detection terminal set 322 includes a positive detection terminal NTC+ and a negative detection terminal NTC-. In an embodiment, the negative control terminal ATOM- and the negative detection terminal NTC- are grounded.

Specifically, the second control terminal set includes a positive control terminal ATOM+ and a negative control terminal ATOM-; and the second detection terminal set includes a positive detection terminal NTC+ and a negative detection terminal NTC-. In an embodiment, the negative control terminal ATOM- and the negative detection terminal NTC- are grounded.

The positive control terminal ATOM+ in the first control terminal set 321 is configured to be connected to the positive control terminal ATOM+ in the second control terminal set, and the negative control terminal ATOM- in the first control terminal set 321 is configured to be connected to the negative control terminal ATOM- in the second control terminal set. The positive detection terminal NTC+ in the first detection terminal set 322 is configured to be connected to the positive detection terminal NTC+ in the second control terminal set, and the negative detection terminal NTC- in the first detection terminal set 322 is configured to be connected to the negative detection terminal NTC- in the second control terminal set.

In an embodiment, the controller 100 is a chargeable controller 100. The atomizer 200 is further configured to charge the controller 100. The atomizer 200 charges the controller 100 through at least some second electrical connection terminals 205 in the plurality of second electrical connection terminals 205 and at least some first electrical connection terminals 32 in the plurality of first electrical connection terminals 32. Specifically, still referring to FIG. 7, the plurality of first electrical connection terminals 32 further include a first charge terminal set 323, and the first charge terminal set 323 includes a positive charge terminal VIN+ and a negative charge terminal VIN-. The plurality of second electrical connection terminals 205 further include a second charge terminal set (not shown in the figure), and the second charge terminal set includes a positive charge terminal VIN+ and a negative charge terminal VIN-. The positive charge terminal VIN+ in the first charge terminal set 323 is configured to be connected to the positive control terminal ATOM+ in the second charge terminal set, and the negative charge terminal VIN- in the first charge terminal set 323 is configured to be connected to the negative charge terminal VIN- in the second charge terminal set.

In an embodiment, as shown in FIG. 7, the positive control terminal ATOM+ and the negative control terminal in the first control terminal set 321 and the second control terminal set are separately arranged connection terminals. The positive detection terminal NTC+ and the negative detection terminal NTC- in the first detection terminal set 322 and the second detection terminal set are separately arranged connection terminals. The positive charge terminal VIN+ and the negative charge terminal VIN- in the first charge terminal set 323 and the second charge terminal set are separately arranged connection terminals. That is, the connection terminals are not shared.

In another embodiment, referring to FIG. 8, FIG. 8 is a schematic structural diagram of a connector from one perspective in the controller shown in FIG. 2. The positive charge terminal VIN+ in the first charge terminal set 323 and the second charge terminal set is a separately arranged connection terminal, and the negative control terminal ATOM- or the negative detection terminal NTC- is used as the negative charge terminal VIN-. That is, the connection terminals are partially shared. In this way, the number of electrical connection terminals on the connector 30 and the second connection portion 203 can be reduced without affecting functions of the controller 100 and the atomizer 200, and the volume of the connector 30 is reduced, thereby saving costs.

The shape of the cross section of the second body portion 204 is approximately the same as that of the first body portion 31. However, one of the second body portion 204 and the first body portion 31 is a male port, and the other is a female port, thereby being convenient for plugging the controller 100 and the atomizer 200.

For ease of description, this application takes the connector 30 as an example for description.

In this application, the extension direction of the first electrical connection terminal 32 is defined as the axial direction X of the controller 100 and the first body portion 31, the axial direction X is the height direction of the controller 100 and the first body portion 31, and the direction perpendicular to the extension direction of the first electrical connection terminal 32 is defined as the radial direction Y of the controller 100 and the first body portion 31.

The shape of the radial cross section of the first body portion 31 is not limited, and may be a symmetric figure or an asymmetric figure. Taking an example in which the shape of the radial cross section of the first body portion 31 is a symmetric figure, the shape of the radial cross section of the first body portion 31 may be a rectangle, and a corner of the rectangle is a rounded corner or a right angle; or, the shape of the radial cross section of the first body portion 31 may be a rhombus, and a corner of the rhombus is a rounded corner or a pointed corner; or, as shown in FIG. 7 or FIG. 8, the shape of the radial cross section of the first body portion 31 is an axisymmetric figure.

In some embodiments, positive and negative terminals in the first control terminal set 321, the first detection terminal set 322 and the first charge terminal set 323 are symmetrically arranged on the first body portion 31. In some other embodiments, positive and negative connection terminals in the first control terminal set 321, the first detection terminal set 322 and the first charge terminal set 323 are irregularly arranged on the first body portion 31. This is not limited herein, provided that electrical-related specifications are met.

In an embodiment, as shown in FIG. 7, the connection terminals arranged on the first body portion 31 are not shared. In addition, to normalize the connector 30 and improve the overall aesthetics, the shape of the radial cross section of the first body portion 31 is set to an axisymmetric figure, and the positive control terminal ATOM+ and the negative control terminal ATOM- are symmetrically arranged along the symmetric axis of the axisymmetric figure; the positive detection terminal NTC+ and the negative detection terminal NTC- are symmetrically arranged along the symmetric axis of the axisymmetric figure; and the positive charge terminal VIN+ and the negative charge terminal VIN- are symmetrically arranged along the symmetric axis of the axisymmetric figure.

In another embodiment, as shown in FIG. 8, the positive charge terminal VIN+ is a separately arranged connection terminal, and the negative control terminal ATOM- or the negative detection terminal NTC- is used as the negative charge terminal VIN-. In addition, to normalize the connector 30 and improve the overall aesthetics, the shape of the radial cross section of the first body portion 31 is set to an axisymmetric figure, and the positive control terminal ATOM+ and the negative control terminal ATOM- are symmetrically arranged along the symmetric axis of the axisymmetric figure; the positive detection terminal NTC+ and the negative detection terminal NTC- are symmetrically arranged along the symmetric axis of the axisymmetric figure; and the positive charge terminal VIN+ is arranged on the symmetric axis of the axisymmetric figure.

In addition, because the connector 30 in this application has a plurality of first electrical connection terminals 32 having different functions, and the second connection portion 203 correspondingly connected to the connector also has a plurality of second electrical connection terminals 205 having different functions, to avoid the misoperation of a user, the first electrical connection terminal 32 and second electrical connection terminal 205 having corresponding functions are not correspondingly electrically connected when the connector 30 is inserted back into the second connection portion 203. Consequently, the controller 100 cannot normally control the atomizer 200 to work, or a circuit fault is generated. As shown in FIG. 7 or FIG. 8, the connector 30 and the second connection portion 203 provided in this application are of an anti-back insertion design. Specifically, the shape of the radial cross section of the first body portion 31 is a non-centrosymmetric figure, and the specific shape thereof may be designed according to actual needs and is not limited herein.

In an embodiment, referring to FIG. 2, the connector 30 is a male port. Specifically, the housing 10 includes the annular side wall 11, the top wall 12 and the bottom wall 13, and the annular side wall 11, the top wall 12 and the bottom wall 13 cooperate to define the first accommodating cavity; and the first body portion 31 is arranged on the outer side of the top wall 12 and is integrally formed and designed with the top wall 12. Alternatively, the first body portion 31 is arranged on the outer side (not shown in the figure) of the bottom wall 13, and is integrally formed and designed with the bottom wall 13.

The first body portion 31 is arranged outside one end of the housing 10, and the first body portion 31 has a plurality of mounting holes 311 with the same number as the first electrical connection terminals 32. The plurality of mounting holes 311 are through holes arranged along the axial direction X of the first body portion 31. The plurality of first electrical connection terminals 32 are correspondingly arranged in the plurality of mounting holes 311 and are electrically connected to the control component 20. When the controller 100 is electrically connected to the atomizer 200, the connector 30 is inserted into the second connection portion 203 of the atomizer 200, so as to implement electrical connection.

Referring to FIG. 4 and FIG. 5, FIG. 4 is a cross-sectional view of the controller shown in FIG. 2 along a line A-A; and FIG. 5 is a structural enlarged view of a region A in FIG. 4. To meet electrical-related design specifications, the first distance H1 between the plurality of first electrical connection terminals 32 and one end of the first body portion 31 facing away from the housing 10 is 2 mm to 5 mm. That is, the plurality of first electrical connection terminals 32 are accommodated in the mounting holes 311, and the distance between the first electrical connection terminal and the plane at which the orifice at one end of the mounting hole 311 facing away from the housing 10 is located is 2 mm to 5 mm.

The apertures of the plurality of mounting holes 311 are 2.0 mm to 3.2 mm. As shown in FIG. 8, five first electrical connection terminals 32 are provided, and the plurality of first electrical connection terminals include a first control terminal set 321, a first detection terminal set 322 and a first charge terminal set 323, where the negative control terminal ATOM- or the negative detection terminal NTC- is used as the negative charge terminal VIN-. Five mounting holes 311 are provided, and the plurality of first electrical connection terminals 32 are correspondingly arranged in the plurality of mounting holes 311 in a one-to-one correspondence manner. In a left-to-right direction, the plurality of first electrical connection terminals 32 are respectively a positive control terminal ATOM+, a positive detection terminal NTC+, a positive charge terminal VIN+, a negative detection terminal NTC-, and a negative control terminal ATOM-. The apertures of the plurality of mounting holes 311 are 2.6 mm, the first hole center distance R1 of the mounting hole 311 configured to accommodate the control terminal and the detection terminal is 4.79 mm, and the second hole center distance R2 of the mounting hole 311 configured to accommodate the detection terminal and the charge terminal is 4.87 mm.

In another embodiment, referring to FIG. 6, FIG. 6 is a structural enlarged view of a region A in another embodiment of a controller provided in this application. The connector 30 is a female port. Specifically, the housing 10 includes the annular side wall 11 and the top wall 12 and the bottom wall 13 that are located at two opposite ends of the annular side wall 11, and the annular side wall 11, the top wall 12 and the bottom wall 13 cooperate to define the first accommodating cavity; and the first body portion 31 is arranged on the inner side of the top wall 12 and is integrally formed and designed with the top wall 12. Alternatively, the first body portion 31 is arranged on the inner side of the bottom wall 13 and is integrally formed and designed with the bottom wall 13.

The first body portion 31 is a groove body arranged in one end of the housing 10. A plane at which the groove opening of the groove body is located is flush with one end of the shell 202. A plurality of first electrical connection terminals 32 are arranged spaced away in the groove body and are fixed to the bottom wall of the groove body. When the controller 100 is electrically connected to the atomizer 200, the second connection portion 203 is inserted into the connector 30 to implement electrical connection.

Specifically, the bottom wall of the groove body is provided with a plurality of fixing holes 312 with the same number as the first electrical connection terminals 32. The plurality of fixing holes 312 are through holes arranged along the axial direction X of the first body portion 31. One ends of the plurality of first electrical connection terminals 32 are fixed to the plurality of fixing holes 312 in a one-to-one correspondence manner and are electrically connected to the control component 20, and the other ends extend into the groove body. The aperture of the fixing hole 312 and the hole center distance of each fixing hole 312 may be the same as the design of the mounting hole 311.

To meet electrical-related design specifications, the second distance H2 between the plurality of first electrical connection terminals 32 and the plane at which the groove opening of the groove body is located is 2 mm to 5 mm. That is, the plurality of first electrical connection terminals 32 are arranged in the groove body and are fixed to the bottom wall 13 of the groove body, and the distance between one end of the plurality of first electrical connection terminals 32 facing away from the bottom wall 13 of the groove body and the plane at which the groove opening is located is 2 mm to 5 mm.

In some embodiments, referring to FIG. 2, the first body portion 31 or the housing 10 is further provided with a buckle structure 40. The buckle structure 40 is configured to fix the second connection portion 203 when the connector 30 is electrically connected to the second connection portion 203, so as to prevent the connector 30 and the second connection portion 203 from being electrically disconnected due to an external force or vibration during the working process of the atomizer 200 and the controller 100, which may affect the normal work of the device.

Referring to FIG. 11, FIG. 11 is a structural exploded view of an embodiment of an atomization component provided in this application. An atomization component 206 provided in this embodiment is an ultrasonic atomization component. Specifically, the atomization component 206 includes a piezoelectric ceramic piece 207 and a metal substrate 208 that are stacked. The atomization component 206 is configured to generate high-frequency vibration when being powered on, so as to atomize an aerosol-forming medium introduced onto the atomization component 206 to generate an aerosol with vector-oriented fine particles, and then, the aerosol is discharged from the vapor outlet 201 along the vapor discharge direction.

Different from the prior art, according to the connector 30, the controller 100 and the atomizer 200 provided in this application, the controller 100 includes a housing 10, a control component 20 and a connector 30; and the atomizer 200 includes a shell 202, an atomization component 206 and a second connection portion 203. Specifically, the connector 30 includes a first body portion 31 and a plurality of first electrical connection terminals 32 arranged on the first body portion 31 and electrically connected to the control component 20. The second connection portion 203 includes a second body portion 204 and a plurality of second electrical connection terminals 205. The plurality of first electrical connection terminals 32 are configured to be electrically connected to the plurality of second electrical connection terminals 205, so as to enable the control component 20 to control the atomization component 206 to work through the plurality of first electrical connection terminals 32 and the plurality of second electrical connection terminals 205, and detect the temperature of the atomizer 200, thereby diversifying functions of an electrical connection interface between the controller 100 and the atomizer 200, and implementing the high system integration degree.

The above descriptions are only implementations of this application, but do not limit the patent scope of this application. Any equivalent structures or equivalent flow transformations made according to the contents of the specification and accompanying drawings of this application for direct or indirect use in other relevant technical fields shall fall within the patent protection scope of this application.

## Claims

1. A connector, applied to an electronic atomization system, comprising a first body portion and a plurality of first electrical connection terminals arranged in the first body portion, wherein the plurality of first electrical connection terminals are configured to be electrically connected to an atomizer and a control component in a controller, so as to enable the control component to control the atomizer to work through the plurality of first electrical connection terminals, and detect the temperature of the atomizer.

2. The connector of claim 1, wherein the plurality of first electrical connection terminals comprise a first control terminal set and a first detection terminal set;
the first control terminal set comprises a positive control terminal and a negative control terminal; and
the first detection terminal set comprises a positive detection terminal and a negative detection terminal.

3. The connector of claim 2, wherein the plurality of first electrical connection terminals further comprise a first charge terminal set, and the first charge terminal set comprises a positive charge terminal and a negative charge terminal.

4. The connector of claim 3, wherein the positive charge terminal and the negative charge terminal are separately arranged charge terminals; or
the positive charge terminal is a separately arranged charge terminal, and the negative control terminal or the negative detection terminal is used as the negative charge terminal.

5. The connector of any one of claims 1 to 4, wherein the shape of the radial cross section of the first body portion is a non-centrosymmetric figure.

6. The connector of claim 1, wherein the first body portion has a plurality of mounting holes, and the plurality of mounting holes are through holes arranged along the axial direction of the first body portion; the plurality of first electrical connection terminals are arranged in the plurality of mounting holes in a one-to-one correspondence manner; and
one end of the plurality of first body portions configured to be connected to the atomizer is a first end, and the distance between each first electrical connection terminal and the end surface of the first end of the first body portion is 2 mm to 5 mm.

7. The connector of claim 1, wherein the first body portion is a groove body, the plurality of first electrical connection terminals are arranged spaced away in the groove body and are fixed to the bottom wall of the groove body, and the distance between the plurality of first electrical connection terminals and the plane at which the groove opening of the groove body is located is 2 mm to 5 mm.

8. A controller, configured to control an atomizer, comprising:
a housing having a first accommodating cavity;
a control component, accommodated in the first accommodating cavity;
a power supply component, electrically connected to the control component; and
a connector, arranged on the housing and electrically connected to the control component, the connector being further configured to be electrically connected to the atomizer,
wherein the connector is the connector of any one of claims 1 to 7.

9. The controller of claim 8, wherein the housing comprises the annular side wall, the top wall and the bottom wall, and the annular side wall, the top wall and the bottom wall cooperate to define the first accommodating cavity; and a first body portion is arranged on the outer side of the top wall and is integrally formed and designed with the top wall, or the first body portion is arranged on the outer side of the bottom wall and is integrally formed and designed with the bottom wall.

10. The controller of claim 8, wherein a buckle structure is arranged on a first body portion or the housing, and the buckle structure is configured to fix the atomizer when the connector is electrically connected to the atomizer.

11. An atomizer, comprising:
a shell having a second accommodating cavity;
an atomization component, accommodated in the second accommodating cavity; and
a second connection portion arranged on the shell and configured to be plugged with a connector, and the second connection portion comprising a second body portion and a plurality of second electrical connection terminals arranged on the second body portion and electrically connected to the atomization component, and the plurality of second electrical connection terminals being configured to be electrically connected to a plurality of first electrical connection terminals on the connector.

12. An electronic atomization system, comprising:
an atomizer, the atomizer being the atomizer of claim 11; and
a controller, the controller being the controller of any one of claims 8 to 10.
